# EUROPÄISCHE PATENTSCHRIFT

(11) **EP 2 794 533 B1**
(45) Veröffentlichungstag und Bekanntmachung des Hinweises auf die Patenterteilung: **24.02.2016**
(21) Anmeldenummer: 12795739.7
(22) Anmeldetag: 28.11.2012
(51) Int. Cl.: C07C 29/80, C07C 29/90, C07C 29/92, C07C 31/22, A47L 9/14

(54) **VERFAHREN ZUR GEWINNUNG VON MIT TRIMETHYLOLPROPAN ANGEREICHERTEN PRODUKTSTRÖMEN AUS DEN NEBENSTRÖMEN DER TRIMETHYLOLPROPANHERSTELLUNG**
PROCESS FOR OBTAINING TRIMETHYLOLPROPANE-ENRICHED PRODUCT STREAMS FROM THE SECONDARY STREAMS OF TRIMETHYLOLPROPANE PREPARATION
PROCÉDÉ POUR L'OBTENTION DE FLUX DE PRODUITS ENRICHIS EN TRIMÉTHYLOLPROPANE À PARTIR DES FLUX SECONDAIRES DE LA PRODUCTION DE TRIMÉTHYLOLPROPANE

(30) Priorität: 23.12.2011 DE 102011122356
(43) Veröffentlichungstag der Anmeldung: 29.10.2014
(73) Patentinhaber: OXEA GmbH, 46147 Oberhausen (DE)
(72) Erfinder: FREY, Guido D., 64560 Riedstadt (DE); STRUTZ, Heinz, 47445 Moers (DE)
(86) Internationale Anmeldenummer: PCT/EP2012/004904
(87) Internationale Veröffentlichungsnummer: WO 2013/091765

(56) Entgegenhaltungen:
- EP-A2- 1 178 030
- WO-A2-01/47850
- GB-A- 1 026 425

## Beschreibung

Die vorliegende Erfindung betrifft ein Verfahren zur Gewinnung von mit Trimethylolpropan angereicherten Produktströmen aus den Nebenströmen der Trimethylolpropanherstellung.

Trimethylolpropan ist ein dreiwertiger Alkohol, der für die Herstellung von Lacken, Polyurethanen und Polyestern, wie beispielsweise von Alkydharzen, von Bedeutung ist. Industriell wird Trimethylolpropan durch Kondensationsreaktion von n-Butyraldehyd mit Formaldehyd nach verschiedenen Varianten hergestellt.

Bei dem sogenannten Hydrierverfahren addiert man zumindest zwei Mole Formaldehyd mit einem Mol n-Butyraldehyd in Gegenwart einer katalytischen Menge eines tertiären Amins über die Zwischenstufe Monomethylolbutyraldehyd zunächst zum Dimethylolbutyraldehyd, der anschließend in einem Hydrierschritt zum Trimethylolpropan umgesetzt wird. Nach dem in WO98/28253 A1 beschriebenen Verfahren wird Formaldehyd mit bis zu einem achtfachen molaren Überschuss eingesetzt. Das erhaltene Reaktionsgemisch aus dem Aldoladditionsschritt wird entweder destillativ oder durch Phasentrennung aufgearbeitet. Bei der destillativen Aufarbeitung werden nicht umgesetzte oder teilumgesetzte Ausgangsverbindungen als flüchtige Komponenten abgezogen und in die Reaktionsstufe zurückgeführt, während das Sumpfprodukt weiter umgesetzt wird. Wird anstelle der destillativen Aufarbeitung das Reaktionsgemisch in einem Phasentrenner in die wässrige und organische Phase geschieden, wird die organische Phase in die Aldoladdition zurückgefahren und die wässrige Phase weiter verarbeitet. Es folgt eine katalytische und/oder thermische Behandlung, um Monomethylolbutyraldehyd in Dimethylolbutyraldehyd zu überführen. Hierbei gebildete Nebenprodukte werden destillativ abgetrennt und das Sumpfprodukt dieser Destillation wird anschließend katalytisch hydriert, um Trimethylolpropan zu gewinnen. Das erhaltene rohe Trimethylolpropan wird anschließend einer Reindestillation unterzogen. Nach Leicht- und Mittelsiederabtrennung erhält man gereinigtes Trimethylolpropan als Zwischenfraktion, während höhersiedende Kondensationsprodukte, in denen Trimethylolpropanäquivalente gebunden sind, als Nachlauf- oder Sumpffraktion anfallen.

Neben dem Hydrierverfahren wird Trimethylolpropan technisch auch über die sogenannte Cannizzaro-Reaktion hergestellt. In einer ersten Reaktionsstufe lässt man dabei n-Butyraldehyd und Formaldehyd unter Zugabe von stöchiometrischen Mengen einer Base zum Dimethylolbutyraldehyd reagieren, das anschließend mit überschüssigem Formaldehyd zum Trimethylolpropan reduziert wird, während gleichzeitig ein Äquivalent Formiat gebildet wird. Üblicherweise wird als Base eine wässrige Lösung einer Alkalimetall- oder Erdalkalimetallverbindung verwendet, beispielsweise Natrium-, Kalium- oder Calciumhydroxid. Da ein Äquivalent Alkalimetall- oder Erdalkalimetallformiat bei dem Cannizzaro-Verfahren als Koppelprodukt anfällt, hängt die Wirtschaftlichkeit dieser Verfahrensvariante auch von den Vermarktungschancen dieses Koppelprodukts ab. Die Aufarbeitung der erhaltenen, wässrigen Reaktionslösung, die Trimethylolpropan, Alkalimetall- oder Erdalkalimetallformiat und überschüssige Base enthält, erfolgt im Allgemeinen durch Extraktion. Nach Neutralisation der überschüssigen Base wird die wässrige Lösung mit einem organischen Lösungsmittel, beispielsweise mit Ethylacetat extrahiert. Die organische Phase wird von der wässrigen Phase, die die Alkalimetall- oder Erdalkalimetallformiate gelöst enthält, getrennt und nach Entfernung des Extraktionsmittels wird Trimethylolpropan durch Destillation gewonnen. Das erhaltene Trimethylolpropan kann weiteren Reinigungsverfahren unterzogen werden. Nach US 5,603,835 wird zunächst aus erhaltenem Trimethylolpropan eine wässrige Lösung hergestellt, die nochmals mit einem organischen Lösungsmittel, beispielsweise mit Methyl-tertiär-butylether, extrahiert wird. Aus der erhaltenen wässrigen Lösung wird Trimethylolpropan mit einer verbesserten Farbzahl von weniger als 100 APHA Einheiten gewonnen.

Gemäß dem aus US 5,948,943 bekannten Verfahren wird die nach der Cannizzaro-Reaktion erhaltene wässrige, rohe Reaktionslösung bei einer solchen Temperatur mit einem geeigneten organischen Lösungsmittel behandelt, dass nur eine flüssige Phase das Extraktionsgefäß verlässt. Bei der anschließenden Abkühlung außerhalb des Extraktionsgefäßes trennt sich die wässrige von der organischen Phase und aus der wässrigen Phase kann Trimethylolpropan mit einer Farbzahl von weniger als 100 APHA isoliert werden.

Es ist ebenfalls bekannt, die Cannizzaro-Reaktion mit einer organischen Base, beispielsweise mit einem tertiären Amin, durchzuführen. Nach der aus WO 97/17313 A1 bekannten Arbeitsweise wird Formaldehyd mit n-Butyraldehyd in Gegenwart von stöchiometrischen Mengen eines tertiären Amins hergestellt, wobei ein Äquivalent des Ammoniumformiats entsteht. Anschließend wird aus dem Rohgemisch Wasser, überschüssiges tertiäres Amin und überschüssiger Formaldehyd abgetrennt und das verbleibende Gemisch erhitzt. Dabei wird das Ammoniumformiat in das tertiäre Amin und Ameisensäure gespalten, wobei das tertiäre Amin und weitere flüchtige Bestandteile abgetrennt werden und es zur Bildung von Trimethylolpropanformiat kommt. Das abgetrennte tertiäre Amin wird entweder in die Cannizzaro-Stufe zurückgeführt oder als Katalysator für die Umesterung des Trimethylolpropanformiats in einer nachgeschalteten Reaktion mit einem zugesetzten niedrigen aliphatischen Alkohol verwendet. Das dabei freigesetzte Trimethylolpropan wird anschließend aus dem Rohprodukt isoliert.

Unabhängig davon, ob die Herstellung von Trimethylolpropan nach dem Hydrierverfahren unter Verwendung katalytischer Mengen eines tertiären Amins, nach dem Cannizzaro-Verfahren mit molaren Mengen eines tertiären Amins und nachfolgender Umesterung des gebildeten Trimethylolpropanformiats oder nach dem Cannizzaro-Verfahren mit molaren Mengen von Alkalimetall- oder Erdalkalimetallhydroxiden und ihrer extraktiven Abtrennung erfolgt, wird das erhaltene rohe Trimethylolpropan einer ein- oder mehrfachen destillativen Reinigung unterzogen, die aufgrund des hohen Siedepunkts im Unterdruck erfolgt. Nach DE 100 58 303 A1 wird die destillative Aufarbeitung des Trimethylolpropans mit einer Ionenaustauscherbehandlung kombiniert, wobei entweder der Aldolisierungsaustrag oder der Hydrieraustrag vor der destillativen Aufarbeitung mit einem stark basischen Ionenaustauscher in Kontakt gebracht wird.

Aus DE 1 768 348 B ist bekannt, zwei verschiedene Aldehyde, beispielsweise Acetaldehyd und Butyraldehyd mit Formaldehyd in einem wässrigen, alkalischen Medium umzusetzen. Das erhaltene Reaktionsgemisch wird zunächst durch Säurezugabe neutralisiert, von suspendierten Feststoffen befreit und anschießend mit sauren und basischen Ionenaustauschern behandelt. EP 1 178 030 A offenbart die Gewinnung von Trimethylolpropan aus einer Destillationsrückstand.

Bei der destillativen Reinigung des rohen Trimethylolpropans fallen neben Schwersiedern und Rückständen auch Fraktionen an, die einen im Vergleich zum Trimethylolpropan geringeren Siedepunkt besitzen. Diese Vorlauffraktionen enthalten neben Leichtsiedern, wie beispielsweise Wasser, Methanol oder Lösungsmittel, auch noch gewisse Mengen an Trimethylolpropan selbst, je nach Trennschärfe in der destillativen Aufarbeitung. Darüber hinaus liegen in der Vorlauffraktion Derivate des Trimethylolpropans vor, die durch vorgeschaltete Reaktionen mit Formaldehyd und Methanol entstanden sind und einen geringeren oder einen mit Trimethylolpropan vergleichbaren Siedepunkt aufweisen.

Unter diesen Derivaten sind besonders formaldehydhaltige Acetale vertreten, die durch das Strukturelement -O-CH₂-O- charakterisiert werden und auch als Formale aufgefasst werden können. Unter den Formalen können folgende lineare und zyklische Formale des Trimethylolpropans strukturell beschrieben werden:

Monozyklisches Formal des Trimethylolpropans:

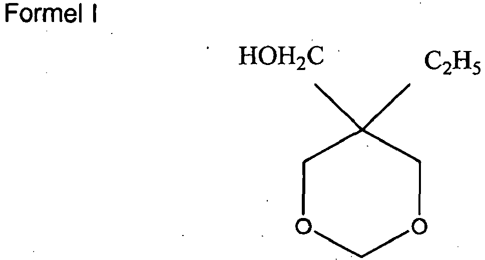

Methyl-(monolineares) Formal des Trimethylolpropans:

Formel II C₂H₅C(CH₂OH)₂CH₂OCH₂OCH₃

Methyl-(bis-lineares) Formal des Trimethylolpropans:

Formel III C₂H₅C(CH₂OH)₂CH₂OCH₂OCH₂OCH₃

Hierbei siedet das monozyklische Formal des Trimethylolpropans (I) bei einer niedrigeren Temperatur als Trimethylolpropan selbst. Die vom Methanol abgeleiteten Formale (II) und (III) haben einen mit Trimethylolpropan vergleichbaren Siedepunkt.

Darüber hinaus sind in der Vorlauffraktion noch 2-Methylbutanol, 2,2-Dimethylpropan-1,3-diol, 2-Ethylpropan-1,3-diol und 2-Ethyl-2-methylpropan-1,3-diol anwesend.

Im Rahmen der Aufarbeitung von schwersiedenden Fraktionen und Rückständen, die bei der destillativen Aufarbeitung des Trimethylolpropans anfallen, werden eine Reihe von Verfahren vorgeschlagen, um insbesondere formaldehydhaltige Acetale zu spalten und Trimethylolpropan freizusetzen, um auf diese Weise die Ausbeute des gesamten Trimethylolpropanherstellprozesses zu verbessern. Nach WO 2004/013074 A1 werden die bei der Trimethylolpropanherstellung erhaltenen schwersiedenden Fraktionen sowie Destillationsrückstände mit Säure behandelt, wobei der Wassergehalt im Reaktionsansatz 20-90 Gew.-% betragen soll. Aus dem säurebehandelten Produkt kann entweder Trimethylolpropan destillativ gewonnen werden oder das behandelte Produkt kann in die Hydrierstufe des Dimethylolbutyraldehyds zum Trimethylolpropan zurückgeführt werden. Die hydrierende Spaltung von linearen oder zyklischen Acetalen in wässrigen Lösungen in Gegenwart eines heterogenen Hydrierkatalysators zu dem gewünschten mehrwertigen Alkohol ist aus DE 198 40 276 A1 bekannt. Das Verfahren erfordert Hydriertemperaturen oberhalb von 160°C, um den schädlichen Einfluss von Formiaten, die besonders bei dem Arbeiten nach dem Cannizzaro-Verfahren noch zugegen sein können, auf die Hydrierleistung des Katalysators zurückzudrängen. Nach der WO 97/01523 A1 kann die Hydriertemperatur zwar erniedrigt werden, doch ist dann ein hohes Gewichtsverhältnis von dem katalytisch wirksamen Metall zu dem zyklischen Formal einzustellen, um eine vertretbare Spaltrate zu erzielen. Ferner werden in der WO 97/01523 A1 und DE 198 40 276 A1 sämtliche Ausführungsbeispiele mit Ruthenium auf Aktivkohle Katalysatoren durchgeführt, um die Ausführbarkeit der offenbarten Verfahren zu belegen. Zur katalytischen Spaltung der formaldehydhaltigen Acetale schlägt der Stand der Technik die Verwendung teurer Edelmetallkatalysatoren bei erhöhter Temperatur vor oder ihren Einsatz in einer vergleichsweisen großen Menge, bezogen auf die formaldehydhaltigen

Acetale. Die hydrierende, katalytische Spaltung kann ebenfalls in Gegenwart einer Säure, beispielsweise in Gegenwart einer niederen Carbonsäure oder sauer reagierender Feststoffe durchgeführt werden.

Da in den Vorlauffraktionen der destillativen Aufarbeitung des Trimethylolpropans erhebliche Mengen an Derivaten vorliegen, in denen Äquivalente des Trimethylolpropans chemisch gebunden sind, ist es wünschenswert, aus diesen Derivaten Trimethylolpropan freizusetzen und zusammen mit noch physikalisch eingemischtem Trimethylolpropan als trimethylolpropanreiche Fraktion zu isolieren, die wieder in den Trimethylolpropanreinigungsprozess zurückgeführt werden kann, so dass die Ausbeute an Trimethylolpropan über den gesamten Herstellprozess verbessert werden kann. Auf diese Weise sollen die Vorlauffraktionen, die bei der destillativen Aufarbeitung im Rahmen der Trimethylolpropanherstellung anfallen, möglichst wirtschaftlich genutzt werden.

Die vorliegende Erfindung betrifft daher ein Verfahren zur Gewinnung von mit Trimethylolpropan angereicherten Produktströmen aus den Vorlauffraktionen, die bei der destillativen Reinigung von Trimethylolpropan anfallen. Es ist dadurch gekennzeichnet, dass man:
(a) die Vorlauffraktionen getrennt oder vereint bei einer Temperatur von 160 bis 280°C und bei einem Druck von 1 bis 30 MPa mit Wasserstoff in Gegenwart eines Hydrierkatalysators und einer aciden Verbindung behandelt; und
(b) das nach Schritt a) erhaltene Reaktionsgemisch in einen mit Trimethylolpropan angereicherten, katalysatorfreien Produktstrom und in einen katalysatorhaltigen Produktstrom destillativ auftrennt,
wobei Trimethylolpropan nach dem Cannizzaro-Verfahren unter Verwendung von Alkalimetall- oder Erdalkalimetallverbindungen oder stöchiometrischer Mengen an Trialkylaminen hergestellt wird oder nach dem Hydrierverfahren in Gegenwart katalytischer Mengen von Trialkylaminen oder Alkalimetall-oder Erdalkalimetallverbindungen produziert wird.

Ausgangsprodukte für das erfindungsgemäße Verfahren sind Stoffströme, die bei der destillativen Reinigung von Trimethylolpropan anfallen und einen geringeren Siedepunkt als Trimethylolpropan aufweisen und als Vorlauffraktionen bezeichnet werden können. Im Allgemeinen sind die Vorlauffraktionen bei Raumtemperatur flüssig.

Die einzelnen Vorlauffraktionen aus der destillativen Aufarbeitung von Trimethylolpropan können vereinigt werden und als Einsatzprodukt nach dem erfindungsgemäßen Verfahren behandelt werden. Es enthält, je nach Destillationsbedingungen, noch physikalisch eingemischtes Trimethylolpropan, im Allgemeinen in einem Bereich von 2 bis 60 Gew.-%, das monozyklische Formal des Trimethylolpropans (Formel I), im Allgemeinen in einem Bereich von 2 bis 70 Gew.-%, das Methyl-(monolineare) Formal des Trimethylolpropans (Formel II) sowie das Methyl-(bis-lineare)Formal des Trimethylolpropans (Formel III), im Allgemeinen in einem Bereich von 0,5 bis 30 Gew.-%, jeweils bezogen auf die Einsatzmasse. Weitere Komponenten in den Vorlauffraktionen sind Leichtsieder, wie Wasser oder über Formaldehyd eingebrachtes Methanol, 2-Methylbutanol, 2,2-Dimethylpropan-1,3-diol, 2-Ethyl-2-methylpropan-1,3-diol sowie 2-Ethylpropan-1,3-diol oder Spuren von Trimethylolpropanacetalen mit den CAS-Nummern 58878-16-3, 115392-09-1, 10441-87-9.

Unabhängig davon, ob Trimethylolpropan nach dem Cannizzaro-Verfahren unter Verwendung von Alkalimetall- oder Erdalkalimetallverbindungen oder stöchiometrischer Mengen an Trialkylaminen hergestellt wird oder nach dem Hydrierverfahren in Gegenwart katalytischer Mengen von Trialkylaminen oder Alkalimetall- oder Erdalkalimetallverbindungen produziert wird, können die Vorlauffraktionen, die bei der destillativen Reinigung von Trimethylolpropan nach dem jeweiligen Herstellverfahren anfallen, gemäß der erfindungsgemäßen Arbeitsweise aufgearbeitet werden.

Da die einzelnen Vorlauffraktionen, die einen niedrigeren Siedepunkt als Trimethylolpropan aufweisen bei Raumtemperatur flüssig sind, ist der Zusatz eines polaren Lösungsmittels nicht zwingend notwendig und die Vorlauffraktionen können direkt und ohne Lösungsmittelzusatz getrennt oder vereinigt katalytisch hydriert werden. Es ist aber auch möglich, die Vorlauffraktionen getrennt oder vereinigt mit einem polaren Lösungsmittel zu versetzen. Als polares Lösungsmittel eignet sich ein niederer C₁-C₅ aliphatischer Alkohol oder C₂-C₁₀-Dialkylether, wie Methanol, Ethanol, Propanol oder Diethylether, oder insbesondere Wasser. Im Allgemeinen stellt man eine Lösung mit einem Gehalt an organischen Komponenten ohne Berücksichtigung des polaren Lösungsmittels von 30 bis 90 Gew.-%, vorzugsweise von 50 bis 90 Gew.-%, bezogen auf die Gesamtmasse her. Geringere Gehalte an organischen Komponenten sind aufgrund des hohen Lösungsmittelanteils nicht zweckmäßig. Üblicherweise wird eine Lösung bei Raumtemperatur hergestellt.

Die Vorlauffraktionen oder daraus erhaltene Lösungen werden anschließend bei erhöhter Temperatur und erhöhtem Druck mit Wasserstoff in Gegenwart eines Hydrierkatalysators und einer aciden Verbindung behandelt. Man arbeitet bei Temperaturen im Bereich von 160 bis 280°C, vorzugsweise 180 bis 230°C und bei Drücken im Bereich von 1 bis 30 MPa, vorzugsweise 5 bis 20 MPa. Bei den anwesenden aciden Verbindungen kann es sich um protische anorganische Säuren, organische Säuren oder um saure Feststoffe handeln. Als protische anorganische Säuren kommen zum Beispiel Phosphorsäure oder Schwefelsäure in Betracht, als organische Säuren niedere Carbonsäuren wie Ameisensäure, Essigsäure, Propionsäure oder die isomeren Buttersäuren.
Ihre Menge wird so bemessen, dass die der Hydrierung zu unterziehende Lösung einen pH-Wert im Bereich von 1 bis 5, vorzugsweise von 2 bis 4 aufweist.

Aufgrund der leichten Abtrennbarkeit ist jedoch das Arbeiten mit sauer reagierenden Feststoffen als acide Verbindung bevorzugt. Als solche Feststoffe eignen sich beispielsweise oxidische Verbindungen wie saures Aluminiumoxid, natürliche oder silikatische Stoffe, wie Mordenit, Montmorillonit oder saure Zeolithe, beispielsweise solche vom Y-Typ, die in technischen Mengen zur Verfügung stehen und beispielsweise bei der katalytischen Spaltung von Rohölen industriell eingesetzt werden. Ihr Zusatz richtet sich nach ihrer Acidität und je 100 Gew.-Teile Lösung werden sie im Allgemeinen in einer Menge von 0,5 bis 2, vorzugsweise von 0,5 bis 1,0 Gew.-Teilen verwendet, wobei die eingesetzten Mengen umso geringer sind je acider der Feststoff ist. Dabei stellt sich in der Lösung im Allgemeinen ein pH-Wert von 1 bis 6, vorzugsweise von 2 bis 4 ein.

Auch kommerziell verfügbare saure Ionenaustauscher, beispielsweise stark saure Ionenaustauscher wie Amberlyst 15, Amberlite IR 120, Amberlyst DPT-1, Dowex Marathon-C, Dowex HCR, Lewatit S 100 oder Naphion oder schwach saure Ionenaustauscher wie Amberlite ICR 86 oder Lewatit CNP können eingesetzt werden. Ihr Zusatz richtet sich nach ihrer Acidität und sie werden im Allgemeinen in einer Menge von 1 bis 20, vorzugsweise von 5 bis 10 Gew.-Teilen, bezogen auf 100 Gew.-Teile Lösung, verwendet, wobei die verwendeten Mengen umso geringer sind je acider der Feststoff ist.

Als Katalysatoren für den Hydrierschritt verwendet man übliche Hydrierkatalysatoren, wobei heterogenen Hydrierkatalysatoren der Vorzug gegeben wird, da sie auf einfache Weise vom Reaktionsgemisch abgetrennt werden können, beispielsweise bei der Suspensionshydrierung durch einfache Filtration. Auch bei fest angeordneten Katalysatoren, beispielsweise bei der Riesel- oder Sumpffahrweise, kann das Reaktionsgemisch vom Hydrierkatalysator leicht separiert werden.

Übliche Hydrierkatalysatoren enthalten als wirksame Komponenten ein Edelmetall aus der Reihe Ru, Rh, Pd oder Pt oder ein Übergangsmetall aus der Reihe Cu, Cr, Co, Ni, Fe und darunter ungeträgerte Vollkatalysatoren wie Nickelkatalysatoren, und insbesondere Raney-Katalysatoren wie Raney-Nickel, oder Chromit-Katalysatoren. Neben ungeträgerten Katalysatoren kommen auch Trägerkatalysatoren zum Einsatz, insbesondere sind für Ru, Rh, Pd oder Pt geeignete Trägermaterialien Aktivkohle, Aluminiumoxid, SiO₂, TiO₂, ZrO₂ sowie Silikate. Die Metallbeladung bei geträgerten Katalysatoren liegt üblicherweise im Bereich von 0,1 bis 15, vorzugsweise von 0,5 bis 10 und insbesondere von 1 bis 5 Gew.-%. Als besonders geeignet haben sich Ru, Pd und Pt auf Aktivkohle erwiesen.

Bei geträgerten Nickelkatalysatoren wird Nickel als katalytisch aktives Metall, im Allgemeinen in einer Menge von etwa 5 bis 70 Gew.-%, vorzugsweise etwa 10 bis etwa 65 Gew.-% und insbesondere etwa 20 bis 60 Gew.-%, jeweils bezogen auf das Gesamtgewicht des Katalysators, aufgebracht. Als Katalysatorträger eignen sich alle herkömmlichen Trägermaterialien, zum Beispiel Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die zum Beispiel der Verbesserung ihrer Hydrieraktivität und/oder ihrer Standzeit und/oder ihrer Selektivität dienen. Derartige Zusatzstoffe sind bekannt, zu ihnen gehören zum Beispiel die Oxide des Natriums, Kaliums, Magnesiums, Calciums, Bariums, Zinks, Aluminiums, Zirconiums und Chroms. Sie werden dem Katalysator im Allgemeinen in einem Anteil von insgesamt 0,1 bis 50 Gew.-Teilen, bezogen auf 100 Gew.-Teile Nickel, zugesetzt.
Die Eignung fester Nickelkatalysatoren für die Hydrierung einer wässrigen formalhaltigen Lösung war nicht zu erwarten, da beispielsweise aus US 5,210,337 bekannt ist, dass Nickelkatalysatoren bei der Hydrierung von Formalen durch anwesendes Formaldehyd geschädigt werden können.

Die Hydrierstufe wird in Gegenwart der aciden Verbindung, die entweder gelöst vorliegt, beispielsweise im Falle von zugesetzten anorganischen Säuren oder niederen organischen Carbonsäuren, oder die als in der Lösung suspendierter Feststoff zugegen ist, kontinuierlich oder diskontinuierlich durchgeführt, beispielsweise an fest angeordneten Katalysatoren nach der Rieselfahrweise oder Sumpffahrweise sowie unter Rühren nach der Suspensionshydrierung.

Bei der kontinuierlichen Fahrweise hat sich eine Katalysatorbelastung V/Vh, ausgedrückt in Durchsatzvolumen pro Katalysatorvolumen und Zeit von 0,1 bis 1 h⁻¹, vorzugsweise von 0,2 bis 0,5 h⁻¹, als zweckmäßig erwiesen. Bei der diskontinuierlichen Verfahrensführung verwendet man, bezogen auf 100 Gew.-Teile Einsatzlösung ohne Berücksichtigung der aciden Verbindung, von 0,1 bis 10, vorzugsweise von 0,25 bis 5 Gew.-Teile an Katalysator.

Nach beendeter Hydrierung wird das flüssige Hydriergut destillativ aufgearbeitet, gegebenenfalls nach Neutralisation mit einer Base. Zunächst werden in einer ersten Destillationseinrichtung das polare Lösungsmittel, falls vor der Hydrierung zugesetzt und Leichtsieder, insbesondere Wasser und Methanol, das durch Hydrierung des bei der Acetalspaltung freigesetzten Formaldehyds entstanden ist, als Kopffraktion abgetrennt. Für die Abtrennung des polaren Lösungsmittels und der Leichtsieder eignen sich übliche Destillationseinrichtungen, wie eine Destillationskolonne mit Sumpfblase, die beispielsweise 2 bis 50 theoretische Böden aufweist, ein Dünnschichtverdampfer, mit oder ohne Kolonnenaufsatz, ein Kurzwegverdampfer oder ein Abdampfbehälter, die üblicherweise bei Sumpftemperaturen von 100 bis 180°C und bei Normaldruck oder zweckmäßigerweise im Unterdruck bis zu 40 hPa betrieben werden.
Es ist ebenfalls möglich, den Druck während der Destillation ausgehend vom Normaldruck bis auf einen sehr niedrigen Druck von 3 hPa stufenweise abzusenken. Die Sumpffraktion aus der ersten Destillationseinrichtung wird anschließend auf eine zweite Destillationseinrichtung gegeben.

In der zweiten Destillationseinrichtung kann man als Kopffraktion einen mit Trimethylolpropan angereicherten, katalysatorfreien Produktstrom gewinnen, der eine Reinheit an Trimethylolpropan von mehr als 96% aufweisen kann und der zudem noch geringe Mengen an Zwischenläufen und Leichtsiedern enthält. Dieser Produktstrom kann in die Reinigungsstufe des Gesamtprozesses für die Herstellung von Trimethylolpropan zurückgeführt werden, zweckmäßigerweise in die Reindestillationsstufe zur Gewinnung von Trimethylolpropan. Die Abtrennung der trimethylolpropanreichen Kopffraktion geschieht ebenfalls in einer üblichen Destillationseinrichtung, wie in einer Destillationskolonne mit Sumpfblase, die eine geeignete Zahl an theoretischen Böden aufweist, in einem Dünnschichtverdampfer, mit oder ohne Kolonnenaufsatz, in einem Kurzwegverdampfer oder in einem Abdampfbehälter, die üblicherweise bei Sumpftemperaturen von 180 bis 280°C und bei Drücken von 3 bis 50, vorzugsweise von 10 bis 25 hPa betrieben werden. Die über den Kolonnenboden abgeführte Sumpffraktion enthält den Katalysator und gegebenenfalls die acide Verbindung, insbesondere dann, wenn in Gegenwart von sauer reagierenden Feststoffen hydriert wird oder wenn bei Verwendung gelöster acider Verbindungen nach der Hydrierung keine Neutralisation mit einer Base erfolgt. Die katalysatorhaltige Sumpffraktion kann, gegebenenfalls nach Zugabe von Frischkatalysator und frischer acider Verbindung in die Hydrierstufe a) des erfindungsgemäßen Verfahren zurückgeführt werden oder zur Hydrierung von schwersiedenden Fraktionen und Rückständen aus der Trimethylolpropanherstellung verwendet werden, beispielsweise in den Verfahren gemäß der DE 10 2011 118 993 A1 oder 10 2011 118 956 A1 zur Gewinnung von Di-Trimethylolpropan und mit Trimethylolpropan angereicherten Produktströmen.

In einer weiteren Ausgestaltung des erfindungsgemäßen Verfahrens können aus dem nach dem Hydrierschritt a) erhaltenen Reaktionsgemisch der Hydrierkatalysator und weitere Feststoffe, falls vorhanden, abgetrennt werden, beispielsweise durch Filtration. Das vom Hydrierkatalysator und gegebenenfalls weiteren Feststoffen befreite Hydriergut wird anschließend wie zuvor beschrieben destillativ aufgearbeitet. Die vorliegende Erfindung betrifft daher ebenfalls ein Verfahren zur Gewinnung von mit Trimethylolpropan angereicherten Produktströmen aus den Vorlauffraktionen, die bei der destillativen Reinigung von Trimethylolpropan anfallen. Es ist dadurch gekennzeichnet, dass man:
(a) die Vorlauffraktionen getrennt oder vereint bei einer Temperatur von 160 bis 280°C und bei einem Druck von 1 bis 30 MPa mit Wasserstoff in Gegenwart eines Hydrierkatalysators und einer aciden Verbindung behandelt;
(b) aus dem nach Schritt a) erhaltenen Reaktionsgemisch Hydrierkatalysator und weitere Feststoffe, falls vorhanden, abtrennt; und
(c) aus dem nach Schritt b) erhaltenen Produkt einen mit Trimethylolpropan angereicherten Produktstrom destillativ gewinnt,
wobei Trimethylolpropan nach dem Cannizzaro-Verfahren unter Verwendung von Alkalimetall- oder Erdalkalimetallverbindungen oder stöchiometrischer Mengen an Tri-alkylaminen hergestellt wird oder nach dem Hydrierverfahren in Gegenwart katalytischer Mengen von Trialkylaminen oder Alkalimetall- oder Erdalkalimetallverbindungen produziert wird.

Wird die Hydrierung in Gegenwart gelöster acider Verbindungen durchgeführt, empfiehlt sich eine Neutralisation mit einer Base, bevor das Hydriergut weiter aufgearbeitet wird. Wie zuvor beschrieben, erfolgt in einer ersten Destillationseinrichtung die Abtrennung von Leichtsiedern als Kopffraktion. Die erhaltene Sumpffraktion wird anschießend auf eine zweite Destillationseinrichtung gegeben, in der eine mit Trimethylolpropan angereicherte Kopffraktion abgezogen wird. Die über den Kolonnenboden abgeführte Sumpffraktion kann anschließend den schwersiedenden Fraktionen und Rückständen aus der Trimethylolpropanherstellung zugemischt werden, die nach dem Verfahren der DE 10 2011 118 953 A1 aufgearbeitet werden können. Die in der ersten und zweiten Destillationseinrichtung angewandten Destillationsbedingungen entsprechend dabei den Bedingungen, die bei der Aufarbeitung des katalysatorhaltigen Hydrierguts eingestellt werden.

In einer weiteren Ausgestaltung des erfindungsgemäßen Verfahrens kann vor der destillativen Aufarbeitung des von Feststoffen befreiten Hydrierguts gegebenenfalls eine Behandlung mit einem Ionenaustauscher erfolgen, beispielsweise entweder nur mit einem basischen oder sauren Ionenaustauscher oder kombiniert in beliebiger Reihenfolge. Man arbeitet bei üblichen Temperaturen im Bereich von 1 bis 100°C, vorzugsweise im Bereich von 20 bis 60°C, und bei Normaldruck.

Falls die Hydrierung in Gegenwart gelöster anorganischer Säuren oder niederer organischer Carbonsäuren erfolgte, wird die Lösung nach Abtrennung des Hydrierkatalyators durch Basenzusatz neutral gestellt. Auch in diesem Falle kann sich die Behandlung mit einem Ionenaustauscher anschließen, und zwar bei üblichen Temperaturen im Bereich von 1 bis 100°C, vorzugsweise im Bereich von 20 bis 60°C, und bei Normaldruck. Durch die Ionenaustauscherbehandlung werden nicht nur die nach Basenzusatz gebildeten Salze abgetrennt sondern darüber hinaus noch weitere Verunreinigungen.

Zu den basischen Ionenaustauschern zählen solche, die primäre, sekundäre, tertiäre oder quartäre Aminogruppen enthalten. Besondere Bedeutung haben Ionenaustauscher auf Polystyrolbasis, die tertiäre Aminogruppen oder quartäre Aminogruppen in der Basenform enthalten, erlangt. Beispiele für schwach- bis starkbasische Ionenaustauscher sind Amberlit IR 45, Dowex 4 oder Dowex Marathon-A. Besondere technische Bedeutung haben makroretikulare Typen, wie Amberlyst A21, Lewatit MP62, Lewatit MP64, Imac A20, Zerolit G, Amberlit IRA93 oder Amberlyst A26 erlangt.

Schwach oder stark saure Ionenaustauscher enthalten beispielsweise die Carboxylat- oder die Sulfonsäuregruppe, die an eine Polymermatrix auf Basis von Styrol-Divinylbenzol-Copolymeren gebunden sind. Die Carboxylatgruppe kann beispielsweise von aromatischen Carbonsäuren oder aliphatischen Carbonsäuren abgeleitet werden und die Sulfonsäuregruppe von aromatischen oder aliphatischen Sulfonsäuren. Ein stark saurer Ionenaustauscher ist beispielsweise Amberlyst 15, Amberlyst DPT-1 oder Dowex Marathon-C.

Man bringt die Lösung in einem geeigneten Reaktor mit dem Ionenaustauscher in Kontakt. Der Ionenaustauscher kann zum Beispiel in einem Rohrreaktor als Festbett angeordnet werden, durch das die Lösung strömt. Das Festbettvolumen und die Größe der Ionenaustauscherpartikel können in weiten Bereichen variiert und so den gewählten Reaktionsbedingungen und den Verfahrensgegebenheiten, wie der gewünschten Strömungsgeschwindigkeit, angepasst werden. Es hat sich bewährt, Raumgeschwindigkeiten im Bereich von 1 bis 10, insbesondere von 5 bis 8 (V Lösung /[V_{Ionenaustauscher} h]) einzuhalten. Es handelt sich hierbei um Richtgrößen, die zweckmäßig zu wählen sind.

Nach einer anderen Ausführungsform der erfindungsgemäßen Arbeitsweise suspendiert man den Ionenaustauscher, der in diesem Fall sehr feinteilig sein kann, in der Lösung. Es ist zweckmäßig, die Suspension ständig zu bewegen, zum Beispiel durch Rühren oder Einleiten eines Gases, beispielsweise Luft oder Stickstoff, um den innigen Kontakt zwischen der flüssigen Phase und dem Ionenaustauscher zu erzielen. Das Massenverhältnis von flüssiger Phase zu Ionenaustauscher kann weitgehend frei und somit den individuellen Erfordernissen entsprechend eingestellt werden. Es hat sich bewährt, je 100 Gew.-Teile Lösung 1 bis 10, vorzugsweise 3 bis 8 Gew.-Teile Ionenaustauscher einzusetzen. Für die Durchführung dieser Verfahrensvariante eignen sich zum Beispiel Rührkessel oder Autoklaven.

Bei dieser Arbeitsweise unterliegt der Ionenaustauscher jedoch einer mechanischen Belastung und für die Vermischung von flüssiger Phase mit dem Ionenaustauscher sind die Bedingungen so einzustelfen, dass ein Abrieb an der Oberfläche der Teilchen oder gar eine mechanische Schädigung der Teilchen vermieden wird.

Die Lösung kann rezirkuliert werden, um durch mehrfache Behandlung der flüssigen Phase die Abtrennung von Verunreinigungen zu vervollständigen. Ebenso ist es möglich, die Adsorption in mehreren Stufen durchzuführen, sowohl absatzweise als auch kontinuierliche Reaktionsführung ist möglich. Die optionale Ionenaustauscherbehandlung eignet sich besonders bei der Aufarbeitung eines wässrigen Hydrierguts.

Nach der optionalen Ionenaustauscherbehandlung des flüssigen Hydrierguts wird das erhaltene Eluat destillativ wie zuvor beschrieben in der zweistufigen Schaltung von Destillationseinrichtungen aufgearbeitet.

Das erfindungsgemäße Verfahren gestattet die wirtschaftliche Verwertung von Vorlauffraktionen, die bei der destillativen Reinigung von Trimethylolpropan anfallen. Durch die Rückführung der daraus gewonnenen trimethylolpropanreichen Produktströme in den gesamten Herstellprozess kann die Anlageneffizienz und die Ausbeute an Trimethylolpropan verbessert werden gegenüber einer Verfahrensführung, bei der die Vorlauffraktionen aus der Trimethylolpropandestillation nicht aufgearbeitet und nicht zurückgeführt werden.

In den nachfolgenden Beispielen wird das erfindungsgemäße Verfahren näher beschrieben. Es ist selbstverständlich nicht auf die wiedergegebene Ausführungsform beschränkt.

### Beispiele

### Beispiel 1

Für die erfindungsgemäße Aufarbeitung der Vorlauffraktionen aus der destillativen Reinigung von Trimethylolpropan kam ein Gemisch zum Einsatz, das folgende gaschromatographisch ermittelte Zusammensetzung (%) aufwies:

| | |
|---|---|
| Vorlauf | 8,4 |
| 2-Methylbutanol | 0 |
| Zwischenlauf I | 0 |
| 2,2-Dimethylpropan-1,3-diol | 1,8 |
| 2-Ethylpropan-1,3-diol | 29,0 |
| 2-Ethyl-2-methylpropan-1,3-diol | 0,1 |
| Monocyclisches Formal (I) | 22,2 |
| Trimethylolpropan | 17,7 |
| Methyl-(monolineares) Formal (II) | 20,7 |
| Hochsieder | 0,1 |

In einem 1 Liter Autoklaven wurden 360 Gramm des organischen Einsatzgemisches mit 240 Gramm Wasser zu einer homogen Lösung vermischt. Man gab 15 Gramm des Zeolith CBV 600 der Firma Zeolyst hinzu sowie 9 Gramm des Hydrierkatalysators. In Versuch 1 wurde ein Ruthenium-auf-Aktivkohle-Katalysator in Pulverform der Firma Johnson Matthey mit einer Metallbeladung von 5 Gew.-% verwendet. Nach Versuch 2 kam der handelsübliche Nickelkatalysator PRICAT Ni52/35 der Firma Johnson Matthey mit einer Nickelbeladung von 52 Gew.-% zum Einsatz. Die Hydrierbedingungen sowie die gaschromatographische Analyse des Hydrierguts sind in der Tabelle 1 zusammengestellt.

**Tabelle 1: Katalytische Hydrierung einer wässrigen Lösung von Vorläufen aus der Trimethylolpropandestillation in Gegenwart von Zeolith CBV 600**

| Reaktionsbedingungen | Versuch 1 | Versuch 2 |
|---|---|---|
| Katalysator | Ru-C | Ni 52/35 |
| Temperatur (°C) | 200 | 200 |
| Druck (MPa) | 8 | 8 |
| Reaktionsdauer (h) | 6 | 6 |

| Gaschromatographisch ermittelte Zusammensetzung (% organischer Anteil, wasserfrei): | | |
|---|---|---|
| Vorlauf | 12,3 | 10,0 |
| 2-Methylbutanol | 2,6 | 1,9 |
| Zwischenlauf I | 0,1 | 0,0 |
| 2,2-Dimethylpropan-1,3-diol | 2,0 | 2,0 |
| 2-Ethylpropan-1,3-diol | 26,0 | 30,0 |
| 2-Ethyl-2-methylpropan-1,3-diol | 0,6 | 0,3 |
| Monocyclisches Formal (I) | 0,1 | 0,4 |
| Trimethylolpropan | 55,0 | 52,7 |
| Methyl-(monolineares) Formal (II) | 1,0 | 2,5 |
| Hochsieder | 0,3 | 0,2 |

### Beispiel 2

Die nach Filtration des Katalysators und des sauren Zeoliths durchgeführte destillative Aufarbeitung eines gemäß Beispiel 1 erhaltenen Hydrierguts zeigte folgende Ergebnisse:

### Erste Destillation an einer 15 Bödenkolonne, Wasser-Leichtsiederabtrennung

| | Einsatz | Kopffraktion | Rückstand |
|---|---|---|---|
| Kopf-Temperatur [°C] | | 65-113 | |
| Sumpf-Temperatur [°C] | | 102-175 | |
| Druck [hPa] | | 1013-3 | |
| Masse [g] | 1034,4 | 697,5 | 336,7* |
| Wasseranteil [%] | 41,8 | 62,0 | nicht bestimmt |

| Gaschromatographisch ermittelte Zusammensetzung (% organischer Anteil, wasserfrei): | | | |
|---|---|---|---|
| Vorlauf | 11,0 | 29,7 | 0,8 |
| 2-Methylbutanol | 2,6 | 10,6 | 0,0 |
| Zwischenlauf I | 0,1 | 0,1 | 0,0 |
| 2,2-Dimethylpropah-1,3-diol | 1,9 | 3,7 | 0,0 |
| 2-Ethylpropan-1,3-diol | 27,1 | 52,4 | 0,3 |
| 2-Ethyl-2-methylpropan-1,3-diol | 0,5 | 0,8 | 0,0 |
| Monocyclisches Formal (I) | 0,3 | 0,5 | 0,0 |
| Trimethylolpropan | 54,3 | 0,1 | 95,4 |
| Methyl-(monolineares) Formal (II) | 0,2 | 0,0 | 0 |
| Hochsieder | 2,0 | 2,1 | 3,5 |

| | | | |
|---|---|---|---|
| *Destillationsverlust: 1034,4 g - 697,5 g - 336,7 g = 0,2 g | | | |

### Zweite Destillation des Rückstands aus der Wasser-Leichtsiederabtrennung (erste Destillation) an einer 15 Bödenkolonne, Trimethylolpropangewinnung, Hochsiederabtrennung

| | Kopffraktion | Rückstand |
|---|---|---|
| Kopf-Temperatur [°C] | 123-135 | |
| Sumpf-Temperatur [°C] | 178-260 | |
| Druck [hPa] | 3 | |
| Masse [g] | 233,7 | 103,0 |

| Gaschromatographisch ermittelte Zusammensetzung (% organischer Anteil, wasserfrei): | | |
|---|---|---|
| Vorlauf | 1,1 | 0,1 |
| 2-Methylbutanol | 0,0 | 0,0 |
| Zwischenlauf I | 0,0 | 0,0 |
| 2,2-Dimethylpropan-1,3-diol | 0,0 | 0,0 |
| 2-Ethylpropan-1,3-diol | 0,6 | 0,0 |
| 2-Ethyl-2-methylpropan-1,3-diol | 0,0 | 0,0 |
| Monocyclisches Formal (I) | 0,0 | 0,0 |
| Trimethylolpropan | 96,3 | 93,4 |
| Methyl-(monolineares) Formal (II) | 0,0 | 0,0 |
| Hochsieder | 2,0 | 6,5 |

Wie das Ergebnis der Hochsiederabtrennung (zweite Destillation) zeigt, kann eine Kopffraktion mit einem Trimethylolpropangehalt von mehr als 96% erhalten werden, die wieder in den Aufarbeitungsprozess bei der Trimethylolpropanherstellung zurückgeführt werden kann.

## Patentansprüche

1. Verfahren zur Gewinnung von mit Trimethylolpropan angereicherten Produktströmen aus den Vorlauffraktionen, die bei der destillativen Reinigung von Trimethylolpropan anfallen, **dadurch gekennzeichnet, dass** man:
(a) die Vorlauffraktionen getrennt oder vereint bei einer Temperatur von 160 bis 280°C und bei einem Druck von 1 bis 30 MPa mit Wasserstoff in Gegenwart eines Hydrierkatalysators und einer aciden Verbindung behandelt; und
(b) das nach Schritt a) erhaltene Reaktionsgemisch in einen mit Trimethylolpropan angereicherten, katalysatorfreien Produktstrom und in einen katalysatorhaltigen Produktstrom destillativ auftrennt,
wobei Trimethylolpropan nach dem Cannizzaro-Verfahren unter Verwendung von Alkalimetall- oder Erdalkalimetallverbindungen oder stöchiometrischer Mengen an Trialkylaminen hergestellt wird oder nach dem Hydrierverfahren in Gegenwart katalytischer Mengen von Trialkylaminen oder Alkalimetall- oder Erdalkalimetallverbindungen produziert wird.

2. Verfahren zur Gewinnung von mit Trimethylolpropan angereicherten Produktströmen aus den Vorlauffraktionen, die bei der destillativen Reinigung von Trimethylolpropan anfallen, **dadurch gekennzeichnet, dass** man:
(a) die Vorlauffraktionen getrennt oder vereint bei einer Temperatur von 160 bis 280°C und bei einem Druck von 1 bis 30 MPa mit Wasserstoff in Gegenwart eines Hydrierkatalysators und einer aciden Verbindung behandelt;
(b) aus dem nach Schritt a) erhaltenen Reaktionsgemisch Hydrierkatalysator und weitere Feststoffe, falls vorhanden, abtrennt; und
(c) aus dem nach Schritt b) erhaltenen Produkt einen mit Trimethylolpropan angereicherten Produktstrom destillativ gewinnt,
wobei Trimethylolpropan nach dem Cannizzaro-Verfahren unter Verwendung von Alkalimetall- oder Erdalkalimetallverbindungen oder stöchiometrischer Mengen an Trialkylaminen hergestellt wird oder nach dem Hydrierverfahren in Gegenwart katalytischer Mengen von Trialkylaminen oder Alkalimetall- oder Erdalkalimetallverbindungen produziert wird.

3. Verfahren gemäß Anspruch 1 oder 2, **dadurch gekennzeichnet, dass** man in Schritt a) bei einer Temperatur von 180 bis 230°C und bei Drücken im Bereich von 5 bis 20 MPa mit Wasserstoff behandelt.

4. Verfahren gemäß einem oder mehreren der Ansprüche 1 bis 3,
**dadurch gekennzeichnet, dass** die destillative Gewinnung der mit Trimethylolpropan angereicherten Produktströme in einer ersten und zweiten Destillationseinrichtung erfolgt.

5. Verfahren gemäß einem oder mehreren der Ansprüche 1 bis 4,
**dadurch gekennzeichnet, dass** der mit Trimethylolpropan angereicherte Produktstrom als Kopffraktion der zweiten Destillationseinrichtung gewonnen wird.

6. Verfahren gemäß einem oder mehreren der Ansprüche 1 bis 5,
**dadurch gekennzeichnet, dass** der in Schritt a) verwendete Hydrierkatalysator ein Hydrierkatalysator mit den wirksamen Komponenten Ru, Rh, Pd oder Pt oder Cu, Cr, Co, Ni oder Fe ist.

7. Verfahren gemäß Anspruch 6, **dadurch gekennzeichnet, dass** der Hydrierkatalysator mit den wirksamen Komponenten Ru, Rh, Pd oder Pt ein Trägerkatalysator mit Aktivkohle, Aluminiumoxid; SiO₂, TiO₂, ZrO₂ oder Silikate als Trägermaterial ist.

8. Verfahren gemäß Anspruch 6, **dadurch gekennzeichnet, dass** der Hydrierkatalysator mit der wirksamen Komponente Nickel ein Trägerkatalysator ist, der Nickel in einer Menge von 5 bis 70 Gew.-%, bezogen auf die Gesamtmasse des Hydrierkatalysators, enthält.

9. Verfahren gemäß Anspruch 8, **dadurch gekennzeichnet, dass** als Trägermaterial Aluminiumoxid, Aluminiumoxidhydrate, Siliciumdioxid, Polykieselsäure, Kieselgur, Kieselxerogele, Magnesiumoxid, Zinkoxid, Zirconiumoxid oder Aktivkohle verwendet wird.

10. Verfahren gemäß Anspruch 8 oder 9, **dadurch gekennzeichnet, dass** der Nickelträgerkatalysator zusätzlich Oxide des Natriums, Kaliums, Magnesiums, Calciums, Bariums, Zinks, Aluminiums, Zirconiums oder Chroms enthält.

11. Verfahren gemäß Anspruch 6, **dadurch gekennzeichnet, dass** der Hydrierkatalysator mit der wirksamen Komponente Nickel ein Vollkatalysator ist.

12. Verfahren gemäß Anspruch 11, **dadurch gekennzeichnet, dass** der nickelhaltige Vollkatalysator Raney-Nickel ist.

13. Verfahren gemäß einem oder mehreren der Ansprüche 2 bis 12,
**dadurch gekennzeichnet, dass** nach dem Schritt b) und vor dem Schritt c) das nach Schritt b) erhaltene Produkt mit einem Ionenaustauscher behandelt wird.

14. Verfahren gemäß Anspruch 13, **dadurch gekennzeichnet, dass** das nach Schritt b) erhaltene Produkt sowohl mit einem basischen als auch mit einem sauren Ionenaustauscher in beliebiger Reihenfolge behandelt wird.

15. Verfahren gemäß einem oder mehreren der Ansprüche 1 bis 14,
**dadurch gekennzeichnet, dass** man die Vorlauffraktionen getrennt oder vereint mit einem polaren Lösungsmittel versetzt und die erhaltene Lösung gemäß Schritt a) mit Wasserstoff behandelt.

16. Verfahren gemäß Anspruch 15, **dadurch gekennzeichnet, dass** man als polares Lösungsmittel einen C₁-C₅-aliphatischen Alkohol, einen C₂-C₁₀-Dialkylether oder Wasser verwendet.

## Claims

1. Process for obtaining trimethylolpropane-enriched product streams from the forerun fractions obtained in the distillative purification of trimethylolpropane, **characterized in that**:
(a) the forerun fractions are treated separately or in combination at a temperature of 160 to 280°C and at a pressure of 1 to 30 MPa with hydrogen in the presence of a hydrogenation catalyst and an acidic compound; and
(b) the reaction mixture obtained after step a) is separated by distillation into a trimethylolpropane-enriched, catalyst-free product stream and a catalyst-containing product stream,
wherein trimethylolpropane is prepared by the Cannizzaro process using alkali metal or alkaline earth metal compounds or stoichiometric amounts of trialkylamines, or is produced by the hydrogenation process in the presence of catalytic amounts of trialkylamines or alkali metal or alkaline earth metal compounds.

2. Process for obtaining trimethylolpropane-enriched product streams from the forerun fractions obtained in the distillative purification of trimethylolpropane, **characterized in that**:
(a) the forerun fractions are treated separately or in combination at a temperature of 160 to 280°C and at a pressure of 1 to 30 MPa with hydrogen in the presence of a hydrogenation catalyst and an acidic compound;
(b) hydrogenation catalyst and further solids, if present, are separated from the reaction mixture obtained after step a); and
(c) a trimethylolpropane-enriched product stream is obtained by distillation from the product obtained after step b),
wherein trimethylolpropane is prepared by the Cannizzaro process using alkali metal or alkaline earth metal compounds or stoichiometric amounts of trialkylamines, or is produced by the hydrogenation process in the presence of catalytic amounts of trialkylamines or alkali metal or alkaline earth metal compounds.

3. Process according to Claim 1 or 2, **characterized in that** treatment with hydrogen in step a) is effected at a temperature of 180 to 230°C and at pressures in the range from 5 to 20 MPa.

4. Process according to one or more of Claims 1 to 3, **characterized in that** the trimethylolpropane-enriched product streams are obtained by distillation in a first and second distillation unit.

5. Process according to one or more of Claims 1 to 4, **characterized in that** the trimethylolpropane-enriched product stream is obtained as the tops fraction of the second distillation unit.

6. Process according to one or more of Claims 1 to 5, **characterized in that** the hydrogenation catalyst used in step a) is a hydrogenation catalyst with the active components Ru, Rh, Pd or Pt, or Cu, Cr, Co, Ni or Fe.

7. Process according to Claim 6, **characterized in that** the hydrogenation catalyst with the active components Ru, Rh, Pd or Pt is a supported catalyst with activated carbon, aluminium oxides, SiO₂, TiO₂, ZrO₂ or silicates as the support material.

8. Process according to Claim 6, **characterized in that** the hydrogenation catalyst with the active component nickel is a supported catalyst containing nickel in an amount of 5 to 70% by weight, based on the total mass of the hydrogenation catalyst.

9. Process according to Claim 8, **characterized in that** the support material used is aluminium oxide, aluminium oxide hydrates, silicon dioxide, polysilicic acid, kieselguhr, silica xerogels, magnesium oxide, zinc oxide, zirconium oxide or activated carbon.

10. Process according to Claim 8 or 9, **characterized in that** the supported nickel catalyst additionally comprises oxides of sodium, potassium, magnesium, calcium, barium, zinc, aluminium, zirconium or chromium.

11. Process according to Claim 6, **characterized in that** the hydrogenation catalyst with the active component nickel is an unsupported catalyst.

12. Process according to Claim 11, **characterized in that** the unsupported nickel catalyst is Raney nickel.

13. Process according to one or more of Claims 2 to 12, **characterized in that**, after step b) and before step c), the product obtained after step b) is treated with an ion exchanger.

14. Process according to Claim 13, **characterized in that** the product obtained after step b) is treated both with a basic and with an acidic ion exchanger in any sequence.

15. Process according to one or more of Claims 1 to 14, **characterized in that** the forerun fractions are admixed separately or in combination with a polar solvent and the resulting solution is treated with hydrogen according to step a).

16. Process according to Claim 15, **characterized in that** the polar solvent used is a C₁-C₅ aliphatic alcohol, a C₂-C₁₀ dialkyl ether or water.

## Revendications

1. Procédé pour la production de flux de produits enrichis en triméthylolpropane à partir des fractions de tête, qui sont produites lors de la purification par distillation de triméthylolpropane, **caractérisé en ce qu'**on
(a) traite les fractions de tête, séparément ou ensemble, à une température de 160 à 280°C et à une pression de 1 à 30 MPa avec de l'hydrogène en présence d'un catalyseur d'hydrogénation et d'un composé acide ; et
(b) sépare par distillation le mélange réactionnel obtenu selon l'étape a) en un flux de produits enrichi en triméthylolpropane, exempt de catalyseur et en un flux de produits contenant du catalyseur,
le triméthylolpropane étant préparé selon le procédé de Cannizzaro avec utilisation de composés de métal alcalin ou de métal alcalino-terreux ou de quantités stoechiométriques de trialkylamines ou étant produit selon le procédé d'hydrogénation en présence de quantités catalytiques de trialkylamines ou de composés de métal alcalin ou de métal alcalino-terreux.

2. Procédé pour la production de flux de produits enrichis en triméthylolpropane à partir de fractions de tête, qui sont produites lors de la purification par distillation de triméthylolpropane, **caractérisé en ce qu'**on
(a) traite les fractions de tête, séparément ou ensemble, à une température de 160 à 280°C et à une pression de 1 à 30 MPa avec de l'hydrogène en présence d'un catalyseur d'hydrogénation et d'un composé acide ;
(b) sépare le catalyseur d'hydrogénation et d'autres solides, s'ils sont présents, du mélange obtenu selon l'étape a) ; et
(c) produit par distillation à partir du produit obtenu selon l'étape b) un flux de produits enrichi en triméthylolpropane,
le triméthylolpropane étant préparé selon le procédé de Cannizzaro avec utilisation de composés de métal alcalin ou de métal alcalino-terreux ou de quantités stoechiométriques de trialkylamines ou étant produit selon le procédé d'hydrogénation en présence de quantités catalytiques de trialkylamines ou de composés de métal alcalin ou de métal alcalino-terreux.

3. Procédé selon la revendication 1 ou 2, **caractérisé en ce qu'**on traite, dans l'étape a), avec de l'hydrogène à une température de 180 à 230°C et à des pressions dans la plage de 5 à 20 MPa.

4. Procédé selon l'une ou plusieurs des revendications 1 à 3, **caractérisé en ce que** la production par distillation des flux de produits enrichis en triméthylolpropane a lieu dans un premier et un deuxième dispositif de distillation.

5. Procédé selon l'une ou plusieurs des revendications 1 à 4, **caractérisé en ce que** le flux de produits enrichi en triméthylolpropane est produit comme fraction de tête du deuxième dispositif de distillation.

6. Procédé selon l'une ou plusieurs des revendications 1 à 5, **caractérisé en ce que** le catalyseur d'hydrogénation utilisé dans l'étape a) est un catalyseur d'hydrogénation présentant les composants actifs Ru, Rh, Pd ou Pt ou Cu, Cr, Co, Ni ou Fe.

7. Procédé selon la revendication 6, **caractérisé en ce que** le catalyseur d'hydrogénation présentant les composants actifs Ru, Rh, Pd ou Pt est un catalyseur supporté présentant du charbon actif, de l'oxyde d'aluminium, du SiO₂, du TiO₂, du ZrO₂ ou des silicates comme matériau support.

8. Procédé selon la revendication 6, **caractérisé en ce que** le catalyseur d'hydrogénation présentant le composant actif nickel est un catalyseur supporté, qui contient le nickel en une quantité de 5 à 70% en poids, par rapport à la masse totale du catalyseur d'hydrogénation.

9. Procédé selon la revendication 8, **caractérisé en ce qu'**on utilise, comme matériau support, de l'oxyde d'aluminium, des oxydes d'aluminium hydratés, du dioxyde de silicium, de l'acide polysilicique, du kieselgur, des xérogels de silice, de l'oxyde de magnésium, de l'oxyde de zinc, de l'oxyde de zirconium ou du charbon actif.

10. Procédé selon la revendication 8 ou 9, **caractérisé en ce que** le catalyseur supporté de nickel contient en outre des oxydes du sodium, du potassium, du magnésium, du calcium, du baryum, du zinc, de l'aluminium, du zirconium ou du chrome.

11. Procédé selon la revendication 6, **caractérisé en ce que** le catalyseur d'hydrogénation présentant le composant actif nickel est un catalyseur plein.

12. Procédé selon la revendication 11, **caractérisé en ce que** le catalyseur plein contenant du nickel est du nickel de Raney.

13. Procédé selon l'une ou plusieurs des revendications 2 à 12, **caractérisé en ce que** le produit obtenu selon l'étape b) est traité, après l'étape b) et avant l'étape c), par un échangeur d'ions.

14. Procédé selon la revendication 13, **caractérisé en ce que** le produit obtenu selon l'étape b) est traité, tant avec un échangeur d'ions basique qu'avec un échangeur d'ions acide, dans un ordre quelconque.

15. Procédé selon l'une ou plusieurs des revendications 1 à 14, **caractérisé en ce qu'**on additionne les fractions de tête, séparément ou ensemble, d'un solvant polaire et on traite la solution obtenue selon l'étape a) par de l'hydrogène.

16. Procédé selon la revendication 15, **caractérisé en ce qu'**on utilise, comme solvant polaire, un alcool aliphatique en C₁-C₅, un C₂-C₁₀-dialkyléther ou de l'eau.
